# EUROPEAN PATENT SPECIFICATION

(11) **EP 0 786 473 B1**
(45) Date of publication and mention of the grant of the patent: **30.03.2005**
(21) Application number: 97100945.1
(22) Date of filing: 22.01.1997
(51) Int. Cl.: C07K 14/47, C07K 14/475, C12S 3/24, A61K 38/17, A61K 38/18, A61K 38/01, A23L 1/305, A23J 3/08, A23L 2/66

(54) **Basic protein composition, basic peptide composition and application thereof**
Basisches Protein Zusammensetzung, basisches Peptid Zusammensetzung und deren Verwendung
Composition de protéine basique, composition de peptide basique et leur application

(30) Priority: 23.01.1996 JP 2845996
(43) Date of publication of application: 30.07.1997
(73) Proprietor: SNOW BRAND MILK PRODUCTS CO., LTD., Sapporo-shi, Hokkaido 065 (JP)
(72) Inventor: Takada, Yukihiro, Kawagoe-shi, Saitama (JP); Aoe, Seiichiro, Sayama-shi, Saitama (JP); Matsuyama, Hiroaki, Kawagoe-shi, Saitama (JP); Kato, Ken, Ohmiya-shi, Saitama (JP); Yamamura, Junichi, Kawagoe-shi, Saitama (JP)
(74) Representative: Boeters, Hans Dietrich, Dr.

(56) References cited:
- EP-A- 0 556 083
- EP-A- 0 573 668
- EP-A- 0 704 218
- TAKADA Y ET AL: "Whey protein stimulated the proliferation and differentiation of osteoblastic MC3T3-E1 cells." BIOCHEMICAL AND BIOPHYSICAL RESEARCH COMMUNICATIONS, (1996 JUN 14) 223 (2) 445-9, XP002073913
- DATABASE WPI Section Ch, Week 9635 Derwent Publications Ltd., London, GB; Class B04, AN 96-350156 XP002073914 & JP 08 165 249 A (SNOW BRAND MILK PROD CO LTD)

## Description

### Field of the Invention

The present invention relates to a milk-derived basic protein composition having action of stimulating osteoblastic proliferation, strengthening bone and inhibiting bone resorption. In addition, the present invention relates to a milk-derived basic peptide composition which is obtained from the milk-derived protein composition by protease degradation and which has action as described above. Further, the present invention also relates to foods, drinks, medicines and feeds which comprise the milk-derived basic protein composition and/or the basic peptide composition.

### Background of the Invention

Accompanying with the prolongation of human life-span, the incidence of metabolic bone diseases, such as osteoporosis, bone fracture, bone pain etc., recently increased. In bone tissue, bone formation and bone resorption are always occurring. While the balance of bone formation and bone resorption is kept in one's youth, bone resorption exceeds bone formation due to various causes as one's age increases(uncoupling). And when bone resorption prolongs for a long duration, bone tissue becomes fragile, which causes metabolic bone diseases, such as osteoporosis, bone fracture, bone pain, etc.. Therefore, if uncoupling can be prohibited, metabolic bone diseases such as osteoporosis, bone fracture, bone pain, etc. are expected to be prevented.

As conventional methods of preventing or treating metabolic bone diseases by inhibiting uncoupling, (1) calcium supplemented diet, (2) light exercise, (3) sunbathing and (4) medicinal therapy, etc. are exemplified. As for calcium supplemented diets, calcium salt, such as calcium carbonate, calcium phosphate, etc., and naturally occurring calcium-containing preparation, such as bovine bone powder, egg shell, fish bone powder, etc. are used. They are, however, not necessarily good for oral intake. As light exercises, jogging or walking may be recommended. However, they are troublesome to a person who becomes weak and quite difficult to an immobilized aged person. Sunbathing is believed to be good for supplement of active form of vitamin D₃ but is not sufficient. As medicinal therapy, 1α-hydroxyvitamin D₃ and/or calcitonin may be used and they are known to be effective for treating osteoporosis. However, they are medicines themselves and can not be used as food sources.

On the other hand, the present inventors found that the fraction obtained from whey protein was effective for strengthening bone (Japanese published unexamined patent application No. 183371 (1992)). Further, the present inventors found that the fraction obtained from the above bone strengthening fraction by treating with ethanol, heating, treating with salts or treating with ultrafiltration membrane was effective for stimulating proliferation of osteoblast and for strengthening bone (Japanese published unexamined patent application No. 176715 (1993), Japanese published unexamined patent application No. 320066 (1993). In addition, the present inventors found that basic protein fraction present in a very small amount in milk had action of stimulating proliferation of osteoblast, of strengthening bone and prohibiting bone resorption (Japanese patent application No. 207509(1995)).

The present inventors found that a basic protein composition obtained from milk-derived basic protein fractions by various kinds of treatment thereof had actions of stimulating osteoblastic proliferation, strengthening bone and inhibiting bone resorption. The inventors, further, found that a basic peptide composition obtained from the above basic protein composition by protease degradation had also actions of stimulating osteoblastic proliferation, strengthening bone and inhibiting bone resorption and, then, accomplished the present invention.

### Summary of the Invention

Accordingly, an object of the present invention is to provide a basic protein composition obtained from milk-derived basic protein fractions, by various kinds of treatment, which has actions of stimulating osteoblastic proliferation, strengthening bone and inhibiting bone resorption. The milk-derived basic protein composition of the present invention has the following properties:
(1) the molecular weight distribution thereof determined by SDS-polyacrylamide electrophoresis is 2,000-24,000 dalton;
(2) the isoelectric point thereof is 7.5-11;
(3) comprising more than 10% of basic amino acids among composing amino acids thereof; and
(4) having actions of stimulating osteoblastic proliferation, strengthening bone and inhibiting bone resorption.

Another object of the present invention is to provide a basic peptide composition obtained from the basic protein composition by protease digestion which has actions of stimulating osteoblastic proliferation, strengthening bone and inhibiting bone resorption. The milk-derived basic peptide of the present invention can be obtained from the above milk-derived basic protein composition by protease digestion.

Further, another object of the present invention is to provide a food, drink, medicine and feed combined with the basic protein composition and/or the basic peptide composition.

### Brief Description of Drawings

Figure 1 represents the results of Test example 1 of a proliferative action thereof on osteoblast.
Figure 2 represents the results of Test example 2 of a stimulative action thereof on collagen synthesis.
Figure 3 represents the results of Test example 3 of a inhibiting action thereof on bone resorption.
Figure 4 represents the results of Test example 4 of an action thereof on strengthening bone.

### Detailed description of the Invention and Preferred Embodiments

The basic protein composition of the present invention is obtained from the basic protein fractions which is present only in a very small amount in milk and has action of stimulating osteoblastic proliferation, strengthening bone and inhibiting bone resorption. That is a milk-derived basic protein composition whose molecular weight distribution determined by SDS-polyacrylamide gel electrophoresis is 2,000-24,000 and whose isoelectric point is 7.5-11. This basic protein contains more than 10% of basic amino acids as composing amino acids.

This basic protein composition can be obtained by the following steps:

After milk or raw material derived from milk is loaded on a cation exchange resin column, a fraction obtained by eluting with an eluent of 0.1-1.0M salt concentration is precipitated (i) by heating over 75°C, (ii) by adding alcohol so that the final concentration thereof will be 10-50% or (iii)by adding salts so that the final concentration thereof will be more than 0.2M; and the supernatant thereof can be recovered.

Alternatively, it can be also obtained by ultrafiltration of a fraction eluted with an eluent of 0.1-1.0M salt concentration with an ultrafiltration membrane cutting off molecular weight more than 30,000 and by recovering the permeated solution.

As milk or milk-derived raw material for obtaining the basic protein composition of the present invention, cow milk, breast milk, goat milk, sheep milk, etc. can be used as they are or reducing milk, skim milk or whey thereof can be also used.

Most of the basic protein composition which is present in a very small amount in milk are lactoferrin and lactoperoxidase, which can be obtained from milk by loading it on a cation exchange resin followed by elution with an eluent of 0.1-1.0M salt concentration. The present inventors found that the lactoferrin or lactoperoxidase did not have any action of stimulating osteoblastic proliferation, strengthening bone or inhibiting bone resorption.

Then, the inventors tried to fractionate a fraction which has stronger action of stimulating osteoblastic proliferation, strengthening bone and inhibiting bone resorption than those of the milk-derived basic protein by the following treatment of the milk-derived basic protein:
(1) the eluted solution of the adsorbed fraction on the above cation exchange resin is heated over 75°C and the precipitate is removed and the supernatant can be recovered. The eluent can be preferably treated by heating at pH6.5-8.0.
(2) to the above eluent, alcohol is added so that the final concentration thereof will be 10-50% and the precipitate is removed and the supernatant can be recovered. As alcohol, cooled ethanol can be preferably used and treatment at pH6.5-9 is preferable.
(3) to the above eluent, salts is added so as to be more than 0.2M of the final concentration thereof and the precipitate is removed and the supernatant can be recovered. On the addition of the salt, the eluent is preferably adjusted at pH6.5-8.0. As the salts, sodium salt such as sodium chloride, potassium salt, ammonium salt, phosphate, bivalent metal salt etc. can be preferably used.
(4) ultra filtrating the above eluent with an ultrafiltration membrane cutting off molecular weight more than 30,000 and the permeated solution can be recovered.

By the treatment of (1)-(4), the basic protein composition having the afore-mentioned actions can be efficiently fractionated. The basic protein composition which is recovered as above and has actions of stimulating osteoblastic proliferation, strengthening bone and inhibiting bone resorption can be usually used as powder obtained by lyophilization and can be usually administered orally.

Alternatively, by an appropriate combination among the above (1)-(4) treatment, that is, heating treatment, alcohol treatment, salt treatment and ultrafiltration treatment, the basic protein composition of the present invention can be obtained. And the basic protein composition has 2,000-24,000 dalton of molecular weight distribution determined by SDS-polyacrylamide gel electrophoresis and 7.5-11 of isoelectric point. Further, it comprises more than 10% of basic amino acids as composing amino acids.

Further, by degradation of this basic protein composition with protease such as pepsin, trypsin, chymotrypsin, pancreatin, etc., the basic peptide composition can be obtained. The basic peptide composition can preferably comprise the basic peptide with less than 10,000 dalton of molecular weight determined by SDS-polyacrylamide gel electrophoresis.

The basic protein composition or the basic peptide composition of the present invention which has actions of stimulating osteoblastic proliferation, strengthening bone and inhibiting bone resorption can be combined with a food, drink, medicine or feed, which can be taken orally to exhibit action of stimulating osteoblastic proliferation, strengthening bone and inhibiting bone resorption. The basic protein composition or the basic peptide composition of the present invention can be taken orally in a form of tablet or in a combined form with milk, juice, yogurt, jelly, bread , ice cream, noodle, sausage etc. For prevention or improvement of osteoporosis or various kinds of metabolic bone diseases, 10-500 mg of the composition can be preferably taken daily. Acute toxicity of the basic protein composition or the basic peptide composition of the present invention was not recognized in rats.

Alternatively, the basic protein composition or the basic peptide composition of the present invention can be more preferably taken orally with calcium salts having good absorptivity. As calcium salt having good absorptivity, calcium chloride, calcium carbonate, calcium lactate, egg shell, milk-derived calcium containing composition etc. can be exemplified.

Since the basic protein composition and the basic peptide composition of the present invention can be taken orally and have actions of stimulating osteoblastic proliferation, strengthening bone and inhibiting bone resorption, they are useful for a food, drink, medicine and feed to prevent or remedy various kinds of metabolic bone diseases, such as bone fracture, rheumatism and arthritis, and osteoporosis.

Then, the present invention will be described in more detail by representing a reference example, examples and test examples as below, which will not limit the scope of the present invention.

### Reference example 1

A column packed with 3,000g of cation exchange resin, sulfonated chitopearl (Fuji-bouseki), was sufficiently washed with deionized water. After loading 300 liter of non-sterilized skim milk on this column at flowing rate of 25ml/min., the column was sufficiently washed with deionized water and 2 l of 0.05M phosphate buffer (pH 7.0) containing 0.1M sodium chloride to elute unadsorbed protein. The basic protein adsorbed on the resin was eluted at 0.1-1.0M sodium chloride gradient using 0.05M phosphate buffer containing 0.1M sodium chloride (pH 7.0) and 0.05M phosphate buffer containing 1.0M sodium chloride (pH 7.0) and recovered. By treating the eluted solution with a reverse osmotic membrane to be desalted and concentrated, followed by lyophilization, powdery basic protein fraction was obtained. By repeating this operation 8 times, 2.83 kg of basic protein fraction was obtained. The molecular weight of this main basic protein fraction was 75,000-85,000 dalton, the isoelectric point thereof was 7.0-8.5. The content of basic amino acids contained in this basic protein fraction was 17.8%.

### Example 1

A column packed with 3,000 g of cation exchange resin, sulfonated chitopearl (Fuji-bouseki), was sufficiently washed with deionized water. After loading 300 l of nonsterilized skim milk on this column at flow rate of 100ml/min., the column was sufficiently washed with deionized water, the basic protein adsorbed on the resin was eluted with 0.02M carbonate buffer solution containing 0.87M sodium chloride (pH 6.7) and was recovered. By treating the eluted solution with a reverse osmotic membrane to be desalted and concentrated, followed by lyophilization, powdery basic protein fraction was obtained. By repeating this operation 8 times, 2.59 kg of basic protein fraction was obtained. Then, 2.59 kg of the basic protein fraction was dissolved in 50 l of deionized water, adjusted to pH 7.5 and 45 l of the solution was heated at 80°C for 10 min., followed by centrifugation (5,000 x g, 10min.) to remove a precipitate, treating with a reverse osmotic membrane to be desalted, concentration and lyophilization to give 530 g of the basic protein composition. The content of basic amino acids contained in this basic protein composition was 14.2%. Further, the molecular weight of this basic protein was 2,000-23,000 dalton and the isoelectric point thereof was 8.0-10.5. Each one liter of the residual solution was heated at 75°C, 90°C and 95°C respectively for 10 min., followed by centrifugation, treating with a reverse osmotic membrane and lyophilization to give 34.2 g, 8.3 g and 6.5 g of basic protein compositions respectively whose content of basic amino acids was 16.5%, 14.0% and 14.0% respectively.

### Example 2

A column packed with 3,000 g of cation exchange resin, sulfonated chitopearl (Fuji-bouseki), was sufficiently washed with deionized water. After loading 300 l of non-sterilized skim milk on this column at flow rate of 100ml/min., the column was sufficiently washed with deionized water, the basic protein adsorbed on the resin was eluted with 0.02M carbonate buffer solution containing 0.87M sodium chloride (pH 6.7) and was recovered. By treating the eluted solution with a reverse osmotic membrane to be desalted and concentrated followed by lyophilization, powdery basic protein fraction was obtained. By repeating this operation 8 times, 2.61kg of basic protein fraction was obtained. Then, 2.61 kg of the basic protein fraction was dissolved in 50 l of deionized water. To 45 l of the solution, 95% ethanol precooled at -20°C was added for the final concentration of ethanol to be 30%, followed by centrifugation (5,000 x g, 10min.) to remove a precipitate and lyophilization to give 453 g of the basic protein composition. The content of basic amino acids containing in this basic protein composition was 13.6%. Further, the molecular weight of this basic protein was 3,000-24,000 dalton and the isoelectric point thereof was 8.0-11.0. To each one liter of the residual solution, ethanol was added to be the final concentration of 10% and 50% respectively, followed by centrifugation and lyophilization to give 39 g and 12 g of basic protein compositions respectively whose content of basic amino acids was 14.6% and 13.6% respectively.

### Example 3

A column packed with 3,000 g of cation exchange resin, sulfonated chitopearl (Fuji-bouseki), was sufficiently washed with deionized water. After loading 300 l of non-sterilized skim milk on this column at flow rate of 100ml/min., the column was sufficiently washed with deionized water, the basic protein adsorbed on the resin was eluted with 0.02M carbonate buffer solution containing 0.87M sodium chloride (pH 6.7) and was recovered. By treating the eluted solution with a reverse osmotic membrane to be desalted and concentrated followed by lyophilization, powdery basic protein fraction was obtained. By repeating this operation 8 times, 2.35 kg of basic protein fraction was obtained. Then, 2.35 kg of the basic protein fraction was dissolved in 50 l of deionized water, adjusted to pH 7.5 and 45 l of the solution was treated with sodium chloride so that the final concentration of sodium chloride was 1.0 M, followed by centrifugation (5,000 x g, 10min.) to remove a precipitate, treating the fraction suspended in 20 l of distilled water with a reverse osmotic membrane to be desalted and concentrated and lyophilization to give 421 g of the basic protein composition. The content of basic amino acids contained in this basic protein composition was 10.7%. Further, the molecular weight of this basic protein was 5,000-23,000 dalton and the isoelectric point thereof was 7.5-10.0. Each one liter of the residual solution was treated with sodium chloride so that the final concentration of sodium chloride was 0.2 M and 2 M respectively, followed by centrifugation, treating with a reverse osmotic membrane and lyophilization to give 2 g and 23 g of basic protein compositions respectively whose content of basic amino acids was 15.4% and 11.0% respectively.

### Example 4

A column packed with 3,000 g of cation exchange resin, sulfonated chitopearl (Fuji-bouseki), was sufficiently washed with deionized water. After loading 300 l of non-sterilized skim milk on this column at flow rate of 100ml/min., the column was sufficiently washed with deionized water, the basic protein adsorbed on the resin was eluted with 0.02M carbonate buffer solution containing 0.87M sodium chloride (pH 6.7) and was recovered. By treating the eluted solution with a reverse osmotic membrane to be desalted and concentrated followed by lyophilization, powdery basic protein fraction was obtained. By repeating this operation 8 times, 2.37 kg of basic protein fraction was obtained. Then, 2.37 kg of the basic protein fraction was dissolved in 50 l of deionized water and 45 l of the solution was treated with an ultrafiltration membrane cutting off molecular weight 500,000(DDS) to give 60 l of permeated solution, followed by treating it with another ultrafiltration membrane cutting off molecular weight 30,000 to be desalted and concentrated to give 6 l of a concentrated solution. Further, treating it with a reverse osmotic membrane and lyophilization to give 430 g of the basic protein composition. The content of basic amino acids contained in this basic protein composition was 15.6%. Further, the molecular weight of this basic protein was 2,000-18,000 dalton and the isoelectric point was 8.0-11.0.

### Example 5

Fifty grams of the basic protein composition heated at 80°C for 10 min. in Example 1 was dissolved in 10 l of distilled water, to which 1% pancreatin(Sigma) was added, followed by reaction at 37°C for 2 hours. After the reaction, the enzyme was inactivated by heating it at 80 C for 10 min. and 48.3 g of the basic peptide composition was obtained. The basic peptide composition had the molecular weight of less than 10,000 dalton and comprised 13.5% of basic amino acids.

### Example 6

Fifty grams of the basic protein composition treated with ethanol(30% the final concentration) in Example 2 was dissolved in 10 l of distilled water, to which 1% pancreatin was added, followed by reaction at 37°C for 2 hours. After the reaction, the enzyme was inactivated by heating it at 80°C for 10 min. and 49.3 g of the basic peptide composition was obtained. The basic peptide composition had the molecular weight of was less than 9,000 dalton and comprised 12.9% of basic amino acids.

### Test example 1

Stimulative action on osteoblastic proliferation of the following samples:
- Sample A:: The basic protein fraction of Reference example 1
- Sample B:: The basic protein composition heated at 75°C for 10 min. in Example 1
- Sample C:: The basic protein composition heated at 80°C for 10 min. in Example 1
- Sample D:: The basic protein composition heated at 90°C for 10 min. in Example 1
- Sample E:: The basic protein composition heated at 95°C for 10 min. in Example 1
- Sample F:: The basic protein composition treated with ethanol (The final concentration of ethanol was 30%) in Example 2
- Sample G:: The basic protein composition treated with ethanol (The final concentration of ethanol was 10%) in Example 2
- Sample H:: The basic protein composition treated with ethanol(The final concentration of ethanol was 50%) in Example 2
- Sample I:: The basic protein composition treated with sodium chloride (The final concentration of sodium chloride was 0.2 M) in Example 3
- Sample J:: The basic protein composition treated with sodium chloride (The final concentration of sodium chloride was 1.0 M) in Example 3
- Sample K:: The basic protein composition treated with sodium chloride (The final concentration of sodium chloride was 2.0 M) in Example 3
- Sample L:: The basic protein composition treated with an ultrafiltration membrane in Example 4
- Sample M:: The basic peptide composition in Example 5
- Sample N:: The basic peptide composition in Example 6

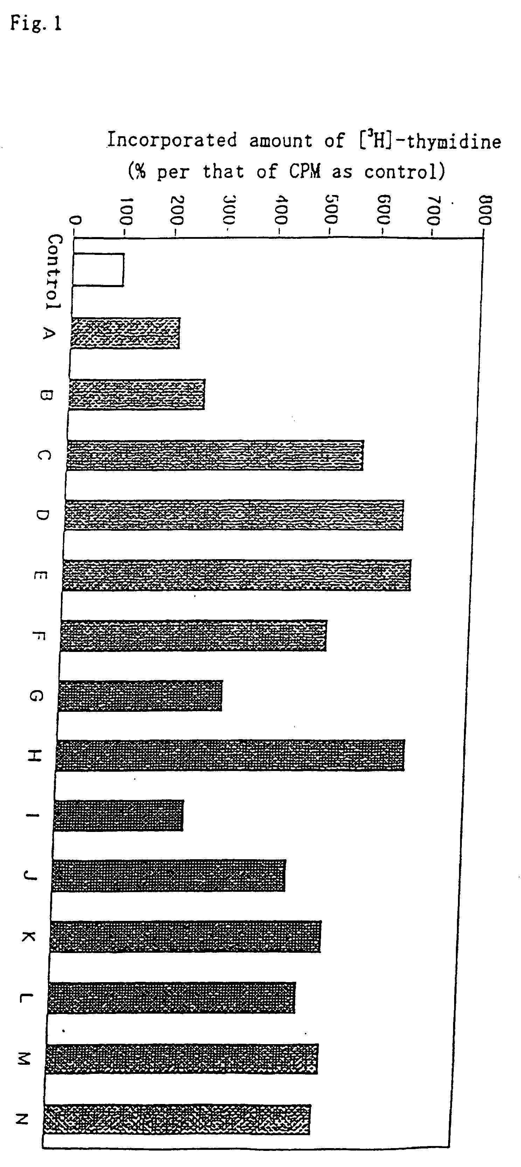

In each well of 96-well plate, 2 x 10⁴cells/ml of osteoblastic cell line MC3T3-E1 in α-modified minimum essential medium (α-MEM) containing 10% bovine fetal serum (Flow Laboratories) was inoculated and cultured in the presence of 5% CO₂ at 37°C for 24 hours, which was used as cultured cell for test. The medium was changed into α-MEM and cultured at 37°C for 16 hours after adding the above sample thereto so that the final concentration thereof was 10 µg/ml. The cells were labeled with [³H]-thymidine, treated by 0.01% Pronase and collected on a glass filter with a cell harvester, to which cocktail for liquid scintillation was added. The incorporated amount of [³H]-thymidine was counted by a liquid scintillation counter and the result was represented in Figure 1.

Comparing the case cultured only with the medium (control), in any case, stimulative activity on osteoblastic proliferation was enhanced, which exhibited that the basic protein fractions and the basic peptide fractions from A to N had an action of stimulating osteoblastic proliferation. In addition, the basic protein compositions of the present invention (sample B-N) was found to have a significant action of stimulating osteoblastic proliferation in comparison with that of the basic protein fraction (sample A) of Reference example.

With respect to another osteoblastic cell line UMR, similar results to the above were obtained.

### Test example 2

According to the method of Woessner [Woessner, J.F., Arch. Biochem. Biophys., vol.93, pp440-447, 1961], action of the same samples A-N as those in Test example 1 on stimulating collagen synthesis was investigated.

In each well of 96-well plate, 2 x 10⁴cells/ml of osteoblastic cell line MC3T3-E1 in α-MEM containing 10% bovine fetal serum (Flow Laboratories) was inoculated and cultured in the presence of 5% CO₂ at 37°C for 24 hours, which was used as cell for test culture. The medium was changed into α-MEM and cultured at 37°C for 3 days after adding the above sample thereto so that the final concentration thereof was 50 µg/ml and the amount of synthesized collagen was determined.

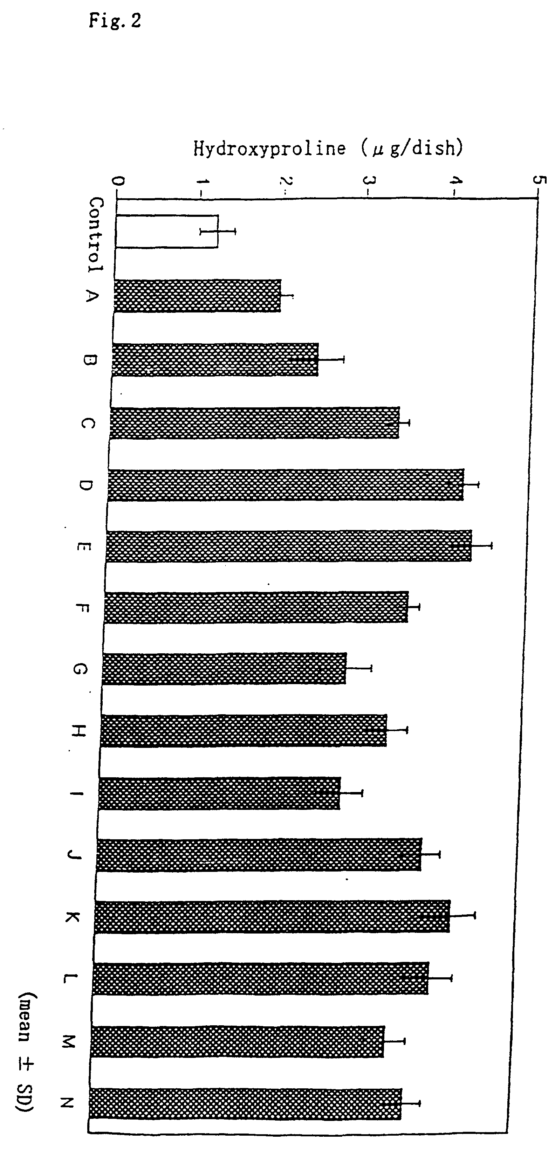

The amount of collagen was analyzed by determining the amount of hydroxyproline using p-dimethyl-aminobenzaldehyde after hydrolyzing punctured cells with 6N hydrochloric acid and the results were represented in Figure 2.

Comparing the case cultured only with the medium (control), in any case, the amount of hydroxyproline increased, which exhibited that the basic protein fractions and the basic peptide fractions from A to N had an action of stimulating collagen synthesis in osteoblast. In addition, the basic protein compositions of the present invention (sample B-N) was found to have a significant action of stimulating collagen synthesis in osteoblast in comparison with that of the basic protein composition (sample A) of Reference example.

### Test example 3

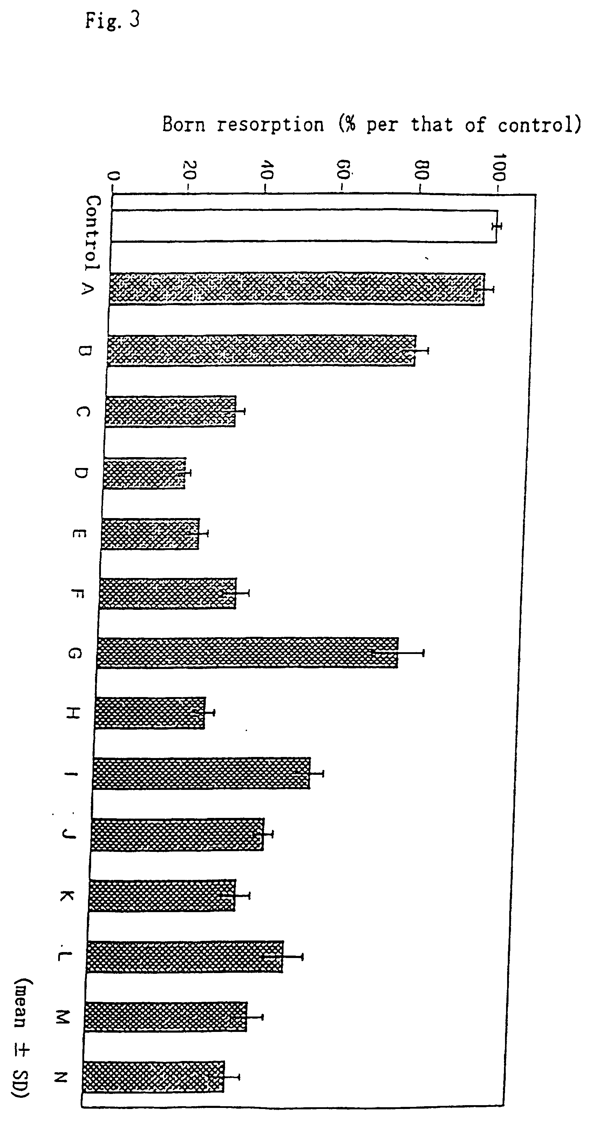

According to the method of Takada [Takada,Y. et al., Bone and Mineral, vol.17, pp.347-359, 1992], action of the same samples A-N as those in Test example 1 on inhibiting bone resorption was investigated. Long bones were obtained from 10-20 days old ICR mice and the whole bone marrow cells obtained osteoclast were obtained by removing soft tissue from the bones and mincing the bones in α-MEM containing 5% bovine fetal serum mechanically. About 2 x 10⁶ of these cells in α-MEM containing 5% bovine fetal serum were placed on a piece of dentine. Several hours after, α-MEM containing 5% bovine fetal serum and a test sample was added to the above, which was cultured for 5 days and bone resorptive activity of osteoclast was investigated. Analysis of bone resorption was carried out by removing cells from a piece of dentinum after cultivation thereof, staining them with hematoxylin dye and measuring the area of bone resorptive cavity by morphometrical analysis with PIAS-LA-555 and the results were represented in Figure 3.

Comparing the case cultured only with the medium(control), a significant bone resorptive action of the basic protein fractions (samples B-N) was observed.

### Test example 4

Action of the following samples on strengthening bone:
- Sample C:: The basic protein composition heated at 80°C for 10 min. in Example 1
- Sample F:: The basic protein composition treated with ethanol (The final concentration of ethanol was 30%) in Example 2
- Sample J:: The basic protein composition treated with sodium chloride (The final concentration of sodium chloride was 1.0 M) in Example 3
- Sample L:: The basic protein composition treated with an ultrafiltration membrane in Example 4
- Sample M:: The basic peptide composition in Example 5
Sample C + milk calcium
Sample F + milk calcium

To the assorted feed represented in Table 1 as a basic feed, 0.3% of each sample was added in place of casein. As milk calcium, one described in Japanese published unexamined patent application No. 306622 (1992) was used in place of calcium contained in a mineral mixture.

**Table 1**

| | |
|---|---|
| Sucrose | 49.3 (g/100g) |
| Casein | 20.0 |
| Corn starch | 15.0 |
| Cellulose | 5.0 |
| Corn oil | 5.0 |
| Vitamin mixture(including choline) | 1.2 |
| Mineral mixture | 4.5 |

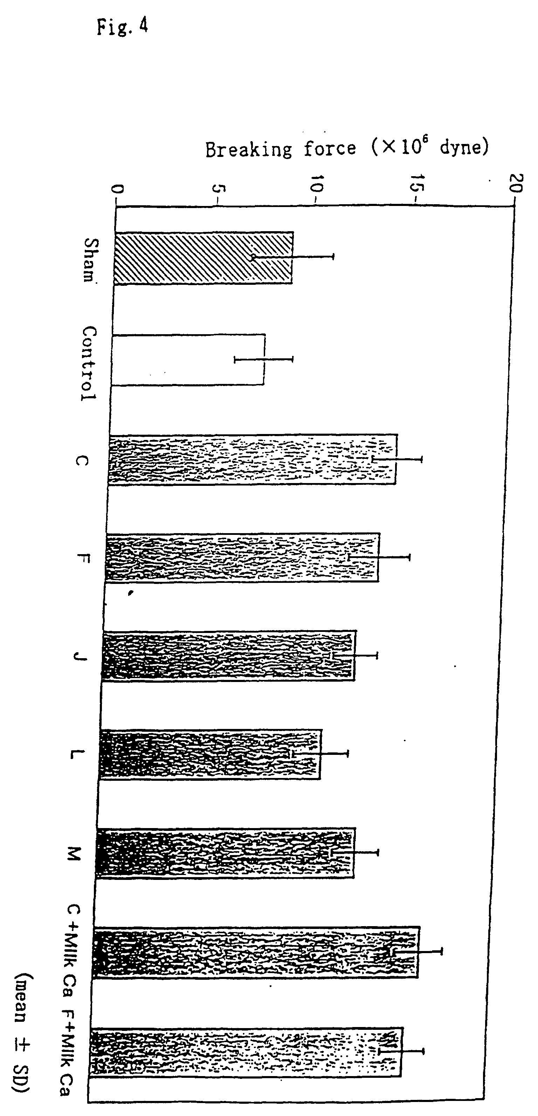

Osteoporotic model rats were made by ovarectomy of 6 weeks old female SD rats and feeding with low calcium diet for two months. Sham rats were also made by sham operation wherein ovary was not removed. Each sample feed was administered in a group consisting of 7 rats for 1 month. Femurs of rats in each test group were taken after the administration of the test feed and the breaking force of the bones were determined with a three-point bending rheolometer (RX-1600, A-techno), whose results were represented in Figure 4. The breaking force of femur of rats in groups administered with the basic protein composition sample C, F, J, L and M was found to be significantly higher than that in the control group according to statistical analysis. From these results, sample C, F, J, L and M of the basic protein composition were found to have an action of strengthening bone. In addition, by combination with milk calcium having good absorptivity, their actions of strengthening bone were found to be augmented.

### Example 7

A drink having actions of stimulating osteoblastic proliferation, strengthening bone and inhibiting bone resorption was prepared by mixing the components represented in Table 2 comprising the basic protein composition heated at 80°C for 10 min in Example 1. (sample C), packing them in a container and sterilizing them by heating.

**Table 2**

| | |
|---|---|
| Mixed isomerized saccharide | 15.0 (weight %) |
| Fruit juice | 10.0 |
| Citric acid | 0.5 |
| Basic protein composition | 0.5 |
| Flavor | 0.1 |
| Calcium | 0.1 |
| Water | 73.8 |

### Example 8

A tablet which has action of stimulating osteoblastic proliferation, strengthening bone and inhibiting bone resorption and comprises the ethanol treated basic protein composition (The final concentration of ethanol was 30%) in Example 2 (sample F) was prepared by mixing the components represented in Table 3 and formulating it under pressure.

**Table 3**

| | |
|---|---|
| Crystalline glucose hydrate | 73.5 (weight %) |
| Basic protein composition | 20.0 |
| Calcium | 5.0 |
| Sugar ester | 1.0 |
| Flavor | 0.5 |

### Example 9

A jelly having action of stimulating osteoblastic proliferation, strengthening bone and inhibiting bone resorption was prepared by mixing the components represented with Table 4 containing ultrafiltrated basic protein composition obtained in Example 4 (sample L), packing it and sterilizing it by heating.

**Table 4**

| | |
|---|---|
| Fractose | 20.0 (weight %) |
| Granulated sugar | 15.0 |
| Millet jelly | 5.0 |
| Agar | 1.0 |
| Basic protein composition | 0.5 |
| Flavor | 0.1 |
| Calcium | 0.1 |
| Water | 58.3 |

### Example 10

A processed cheese which has action of stimulating osteoblastic proliferation, strengthening bone and inhibiting bone resorption and comprises the sodium chloride treated basic protein composition (The final concentration of sodium chloride was 1.0 M) in Example 3 (sample J) was prepared by mixing the contents represented in Table 5 and sterilizing it at 85°C.

**Table 5**

| | |
|---|---|
| Gouda cheese | 43.0 (weight %) |
| Cheddar cheese | 43.0 |
| Sodium citrate | 2.0 |
| Basic protein composition | 0.5 |
| Milk derived calcium | 1.0 |
| Water | 10.5 |

### Example 11

A cracker which has action of stimulating osteoblastic proliferation, strengthening bone and inhibiting bone resorption and containing sodium chloride treated basic protein (The final concentration of sodium chloride was 1.0 M) in Example 3 (sample J) by mixing the components represented in Table 3 was prepared by mixing the contents represented in Table 6, making dough and baking it.

**Table 6**

| | |
|---|---|
| Wheat powder | 50.0 (weight %) |
| Sugar | 20.0 |
| Sodium chloride | 0.5 |
| Margarine | 12.5 |
| Egg | 12.1 |
| Water | 2.5 |
| Sodium bicarbonate | 0.1 |
| Ammonium bicarbonate | 0.2 |
| Calcium carbonate | 0.5 |
| Basic protein composition | 1.2 |

### Example 12

A feed for dog which has action of stimulating osteoblastic proliferation, strengthening bone and inhibiting bone resorption and comprises the basic protein composition heated at 80°C for 10min. in Example 2 (sample C) was prepared by mixing the components represented in Table 7.

**Table 7**

| | |
|---|---|
| Soy bean cake | 12.0 (weight %) |
| Skim milk powder | 14.0 |
| Soy bean oil | 4.0 |
| Corn oil | 2.0 |
| Palm oil | 28.0 |
| Corn starch | 15.0 |
| Wheat powder | 8.0 |
| Wheat bran | 2.0 |
| Vitamin mixture | 9.0 |
| Mineral mixture | 2.0 |
| Cellulose | 3.0 |
| Basic protein composition | 1.0 |

## Claims

1. A milk-derived basic protein composition having the following properties:
(1) the molecular weight distribution thereof determined by SDS-polyacrylamide gel electrophoresis is 2,000-24,000 dalton;
(2) the isoelectric point thereof is 7.5-11;
(3) it comprises more than 10 % of basic amino acids as composing amino acids; and
(4) it has actions of stimulating osteoblastic proliferation, strengthening bone and inhibiting bone resorption.

2. A process to obtain the milk-derived basic protein according to claim 1 which comprises the following steps:
loading milk or milk-derived raw material on a cation exchange resin;
eluting the adsorbed fraction thereon with an eluent of 0.1-1.0 M salt concentration;
heating the fraction at higher than 75°C; and
removing the precipitate and recovering the supernatant.

3. A process to obtain the milk-derived basic protein according to claim 1 which comprises the following steps:
loading milk or milk-derived raw material on a cation exchange resin;
eluting the adsorbed fraction thereon with an eluent of 0.1-1.0 M salt concentration;
adding ethanol so that the final concentration thereof will be 10-50% ; and
removing the precipitate and recovering the supernatant.

4. A process to obtain the milk-derived basic protein according to claim 1 which comprises the following steps:
loading milk or milk-derived raw material on a cation exchange resin;
eluting the adsorbed fraction thereon with an eluent of 0.1-1.0 M salt concentration;
adding salts so that the final concentration thereof will be more than 0.2 M; and
removing the precipitate and recovering the supernatant.

5. A process to obtain the milk-derived basic protein according to claim 1 which comprises the following steps:
loading milk or milk-derived raw material on a cation exchange resin;
eluting the adsorbed fraction thereon with an eluent of 0.1-1.0 M salt concentration;
ultra filtrating it with a ultrafiltration membrane cutting off more than 30,000 ; and
recovering the permeated solution.

6. The milk-derived basic peptide composition which is obtained by protease digestion of the milk-derived basic protein composition according to claim 1.

7. The basic peptide composition according to claim 6 which comprises basic peptides of the molecular weight less than 10000 dalton determined by SDS-polyacrylamide gel electrophoresis.

8. A drink, food, medicine or feed combined with milk-derived basic protein composition and/or milk-derived basic peptide composition according to any one of claims 1 or 6.

## Patentansprüche

1. Aus Milch stammende basische Protein-Zusammensetzung mit den folgenden Eigenschaften:
(1) die durch SDS-Polyacrylamid-Gelelektrophorese bestimmte Molekulargewichtsverteilung derselben ist 2000-24000 Dalton;
(2) der isoelektrische Punkt derselben ist 7,5-11;
(3) sie umfasst mehr als 10% basische Aminosäuren als bildende Aminosäuren; und
(4) sie hat Wirkungen, die Vermehrung der Osteoblasten zu stimulieren, Knochen zu festigen und Knochenresorption zu hemmen.

2. Verfahren, um das aus Milch stammende basische Protein nach Anspruch 1 zu erhalten, welches die folgenden Schritte umfasst:
Laden von Milch oder aus Milch stammendem Rohmaterial auf ein Kationenaustauschharz;
Eluieren der daran adsorbierten Fraktion mit einem Eluierungsmittel mit 0,1-1,0 M Salzkonzentration;
Erhitzen der Fraktion auf mehr als 75° C; und
Abtrennen des Präzipitats und Gewinnen des Überstands.

3. Verfahren, um das aus Milch stammende basische Protein nach Anspruch 1 zu erhalten, welches die folgenden Schritte umfasst :
Laden von Milch oder aus Milch stammendem Rohmaterial auf ein Kationenaustauschharz;
Eluieren der daran adsorbierten Fraktion mit einem Eluierungsmittel mit 0,1-1,0 M Salzkonzentration;
Zugeben von Ethanol, so dass die Endkonzentration desselben 10-50% sein wird; und
Abtrennen des Präzipitats und Gewinnen des Überstands.

4. Verfahren, um das aus Milch stammende basische Protein nach Anspruch 1 zu erhalten, welches die folgenden Schritte umfasst:
Laden von Milch oder aus Milch stammendem Rohmaterial auf ein Kationenaustauschharz;
Eluieren der daran adsorbierten Fraktion mit einem Eluierungsmittel mit 0,1-1,0 M Salzkonzentration;
Zugeben von Salzen, so dass die Endkonzentration derselben mehr als 0,2 M sein wird; und
Abtrennen des Präzipitats und Gewinnen des Überstands.

5. Verfahren, um das aus Milch stammende basische Protein nach Anspruch 1 zu erhalten, welches die folgenden Schritte umfasst:
Laden von Milch oder aus Milch stammendem Rohmaterial auf ein Kationenaustauschharz;
Eluieren der daran adsorbierten Fraktion mit einem Eluierungsmittel mit 0,1-1,0 M Salzkonzentration;
Ultrafiltrieren von dieser mit einer Ultrafiltrationsmembran mit einer Ausschlussgröße von mehr als 30000; und
Gewinnen der permeierten Lösung.

6. Aus Milch stammende basische Peptid-Zusammensetzung, welche durch Proteaseverdau der aus Milch stammenden basischen Protein-Zusammensetzung nach Anspruch 1 erhalten wird.

7. Basische Peptid-Zusammensetzung nach Anspruch 6, welche basische Peptide mit einem Molekulargewicht von weniger als 10000 Dalton, bestimmt durch SDS-Polyacrylamid-Gelelektrophorese, umfasst.

8. Getränk, Nahrung, Arznei oder Futter, kombiniert mit aus Milch stammender basischer Protein-Zusammensetzung und/oder aus Milch stammender basischer Peptid-Zusammensetzung nach einem der Ansprüche 1 oder 6.

## Revendications

1. Composition de protéine basique dérivée du lait ayant les propriétés suivantes :
(1) répartition du poids moléculaire de la composition déterminée par électrophorèse sur gel de polyacrylamide en présence de SDS de 2000 à 24000 daltons ;
(2) point isoélectrique de la composition compris entre 7,5 et 11 ;
(3) la composition comprend plus de 10 % d'acides aminés basiques en tant qu'acides aminés constitutifs ; et
(4) la composition agit sur la stimulation de la prolifération des ostéoblastes, sur le renforcement osseux et sur l'inhibition de la résorption osseuse.

2. Procédé visant à obtenir la protéine basique dérivée du lait selon la revendication 1 qui comprend les étapes suivantes :
charger le lait ou le matériau brut dérivé du lait sur une résine échangeuse de cations ;
éluer la fraction adsorbée sur la résine avec un éluant dont la concentration en sel est comprise entre 0,1 et 1,0 M ;
chauffer la fraction à plus de 75°C ; et
éliminer le précipité et récupérer le surnageant.

3. Procédé visant à obtenir la protéine basique dérivée du lait selon la revendication 1 qui comprend les étapes suivantes :
charger le lait ou le matériau brut dérivé du lait sur une résine échangeuse de cations ;
éluer la fraction adsorbée sur la résine avec un éluant dont la concentration en sel est comprise entre 0,1 et 1,0 M ;
ajouter de l'éthanol de sorte que la concentration finale en éthanol soit comprise entre 10 et 50 % ; et
éliminer le précipité et récupérer le surnageant.

4. Procédé visant à obtenir la protéine basique dérivée du lait selon la revendication 1 qui comprend les étapes suivantes :
charger le lait ou le matériau brut dérivé du lait sur une résine échangeuse de cations ;
éluer la fraction adsorbée sur la résine avec un éluant dont la concentration en sel est comprise entre 0,1 et 1,0 M ;
ajouter des sels de sorte que la concentration finale en sel dépasse 0,2 M ; et
éliminer le précipité et récupérer le surnageant.

5. Procédé visant à obtenir la protéine basique dérivée du lait selon la revendication 1 qui comprend les étapes suivantes :
charger le lait ou le matériau brut dérivé du lait sur une résine échangeuse de cations ;
éluer la fraction adsorbée sur la résine avec un éluant dont la concentration en sel est comprise entre 0,1 et 1,0 M ;
ultra-filtrer la fraction éluée à l'aide d'une membrane d'ultrafiltration extrayant les poids moléculaires dépassant 30000 ; et
récupérer la solution filtrée.

6. Composition de peptide basique dérivée du lait obtenue par digestion protéasique de la composition de protéine basique dérivée du lait selon la revendication 1.

7. Composition de peptide basique selon la revendication 6 qui comprend des peptides basiques d'un poids moléculaire inférieur à 10000 daltons déterminé par électrophorèse sur gel de polyacrylamide en présence de SDS.

8. Boisson, nourriture, médicament ou aliments combinés avec une composition de protéine basique dérivée du lait et/ou une composition de peptide basique dérivée du lait selon la revendication 1 ou 6.
